# EUROPEAN PATENT SPECIFICATION

(11) **EP 3 547 972 B1**
(45) Date of publication and mention of the grant of the patent: **18.09.2024**
(21) Application number: 17878684.4
(22) Date of filing: 05.12.2017
(51) Int. Cl.: A61F 5/01, B29C 51/00, A61F 2/50, A61F 13/04

(54) **THERMOPLASTIC ORTHOPEDIC DEVICES**
THERMOPLASTISCHE ORTHOPÄDISCHE VORRICHTUNGEN
DISPOSITIFS ORTHOPÉDIQUES THERMOPLASTIQUES

(30) Priority: 05.12.2016 IL 24940016
(43) Date of publication of application: 09.10.2019
(73) Proprietor: Cassit Orthopedics Ltd., 6608026 Tel Aviv (IL)
(72) Inventor: ISH CASSIT, Tamar, Tel Aviv 6608026 (IL)
(74) Representative: Weickmann & Weickmann PartmbB
(86) International application number: PCT/IL2017/051319
(87) International publication number: WO 2018/104940

(56) References cited:
- EP-A2- 0 686 380
- WO-A2-2012/032226
- WO-A2-2014/030143
- US-A1- 2007 016 323
- US-A1- 2008 010 856
- US-A1- 2008 010 856
- US-A1- 2012 073 584
- US-A1- 2012 073 584
- US-A1- 2016 242 965
- US-A1- 2016 242 965
- US-A1- 2016 286 897

## Description

### TECHNOLOGICAL FIELD

The present disclosure concerns orthopedic and assistive devices, more particularly splint and prosthesis devices made of thermoplastic materials.

### BACKGROUND ART

References considered to be relevant as background to the presently disclosed subject matter are listed below;
- WO2012/032226 A2, which discloses the preamble of claim 1.
   - US 8,951,217
   - US 2012/0203154

Acknowledgement of the above references herein is not to be inferred as meaning that these are in any way relevant to the patentability of the presently disclosed subject matter.

### BACKGROUND

Orthopedic devices, such as splints and prostheses, are widely used for various purposes. Such devices are often utilized in order to provide support or limit the movement of an injured and/or disabled limb, either as emergency first response treatment to an injured individual or as a part of prolonged treatment and/or rehabilitation period of an injured and/or disabled limb. In order to provide support to the limb during its various stages of recovery or prevention of its deterioration, and due to the variability of dimensions of the limbs in different patients, many types of splints and prostheses exist, varying in their shape, size, geometrical and spatial features, etc.

Splints and prostheses may also be utilized in physiotherapy, O&P (orthosis and prosthetic), occupational therapy, and after surgery and/or micro-surgery fixation. For example, hand splints and assistive devices are suitable for individuals with physical disabilities and injuries such as individuals suffering from common upper body disabilities, resulting mainly from cerebral palsy, stroke, spinal cord injuries, head injuries, traumatic injuries (fractures, sprains) and other diseases. Another significant segment of this market consists of people with RSI (Repetitive Strain Injury) or CTS (Carpal Tunnel Syndrome). Prosthetics are mostly suitable for individuals with amputated limbs or fingers, or partially or fully paralyzed limbs or fingers. Similarly, lower extremities splints and prostheses have various configurations for various purposes.

It is generally understood that individuals suffering from cerebral palsy (CP) often tend to have abnormal muscle tone in one or more of their extremities and experience difficulties in flexing their muscles resulting in deformities and general movement disabilities. CP Splints are used to support affected limbs, maintain motor skills, prevent shortening of range of motion and therefore prevent deformities and/or further deterioration. Functional splints, for example, bring the hand to a functional position and allows the optimal use of strength of the fingers and grasping of various tools.

As may be appreciated, in order to obtain proper fitting of the splint to an individual, splints and prostheses are often customized to the individual's needs and limb features. Such customization is often time consuming, expensive and requires involvement of specifically trained practitioners.

To allow practitioners to adapt a splint or a prosthesis to specific requirements, thermoplastic materials are often used. Such materials have a melting point allowing a practitioner to soften the material, cut and sculpt it to the required shape suitable for the patients' needs. The shape is typically cut to suit the patient's anatomy and type of disability, and is finally chilled for fixating the resulting splint. Commonly used thermoplastic materials, such a polycaprolactone, used to prepare such splints often have low durability and a relatively low melting point, resulting in unwanted deformation when used in hot climates or otherwise exposed to elevated temperatures which individuals may be exposed to during routine daily activities.

Further, although such solutions allow practitioners a wide degree of variability, such solutions require a high degree of skill and access to specialized facilities and several types of accessories. However, there are many territories and countries that lack such means, leaving such solutions falling short in assisting a large and significant part of those populations.

A different approach involves the use of pre-cut shapes of different materials, suit an average body type and shape, which are used to hold a limb in a general position. As may be appreciated, these generic solutions provide poor orthopedics support and therefore are only suitable to a limited range of injuries and/or disabilities.

Practitioners in the field deal with conflicting limitations: (1) implementing structures that bring the limb to a position that allows and encourages the functioning of the limb; and (2) due to the relatively poor mechanical properties of the material from which the splint is made, providing such support over large portions of the limb, including areas that are vital for movement, often need to be covered by the orthopedic device, and therefore further or unnecessarily limiting the movement of the limb and/or impairs grasping and use of tools and accessories, and/or resulting high weight of the splint and thus difficulty in using it. For example, a cock-up splint can bring the hand to an optimal functional position, but while wearing it, it is impossible to properly hold a cell phone, a bag, a hammer or a bottle, because the splint covers the grip area. Another example is walking splints, which are often heavy and bulky. Thus, the overall weight of the device needs also to be taken into consideration.

In addition, many practitioners indicate they avoid making splints because they lack the time and/or the knowledge and/or the means required to prepare splints and/or prosthetics, due to the tiresome and time-consuming process described above.

Thus, there is a long-standing need for a solution that will provide maximum tailoring and flexibility to a practitioner in minimum shaping time and equipment, while providing proper support to the injured limb.

### GENERAL DESCRIPTION

The invention is defined in the appended claims.

The present disclosure concerns orthopedic devices, e.g. splints and prostheses, that can be easily and quickly customized to the patient's anatomical features, without using expensive equipment and without requiring specialization in splinting, orthosis and prosthetics for the preparation of the device for the patient. Further, the devices of this disclosure are mechanically stable, such that once shaped and customized into the desired spatial configuration and hardened, the device provides proper support to the injured or disabled limb.

Further, the devices of this disclosure are formable and shapeable without the need of special equipment, thus making the devices suitable for providing treatment to populations in poor or developing countries, which constitute the vast majority of the world population living with disabilities, as well as in disaster areas.

Thus, in a first aspect, this disclosure provides a formable orthopedic device having at least one first configuration and at least one second configuration, the second configuration being adapted to provide support to at least one body limb, the device being made of a thermoplastic polymeric material selected to have an elastic modulus of at least about 1500 MPa when measured according to ASTM D638, a glass transition temperature (Tg) of between about 65°C and about 120°C, and elongation to break of at least 75% when measured according to ASTM D638, the device being capable to be formed into the second configuration by heating the device to said Tg.

The devices of this disclosure are ***orthopedic devices**,* namely, devices that have an orthopedic or occupational therapy or physical therapy purpose, typically providing support to a limb, limiting the movement of a joint and/or enabling movement and functioning of the injured and/or disabled or amputated limb, fixating the relative movement and/or position of one body part with respect to the other, training muscular movement, changing muscle tone, etc. The orthopedic devices of this disclosure are, but not limited to, a splint, a prosthesis, a limb encasing device, an assistive device, etc.

In some embodiments, the splint may be selected from a functional splint, rest/resting splint, finger splint, cock-up splint, thumb splint, Thumb Spica splint, thumb spica fixation, dorsal extension-block splint, thumb-wrist splint, armpit splint, short arm splint, forearm splint, arm splint, wrist splint, volar wrist splint, shoulder splint, cerebral palsy (CP) splint, elbow splint, elbow immobilizer, dynamic splint, walking splint, knee-ankle-foot orthosis (KAFO), Knee Orthosis (KO), ankle-foot orthosis (AFO), hip-knee-ankle-foot orthosis (HKAFO), ground reaction ankle-foot orthosis (GRAFO), supra-malleolar AFO (SMAFO), knee splint, ankle splint, ankle stirrup, thigh splint, calf splint, shin splint, hip splint, back splint, leg splint, neck splint, cervical collar, erbs palsy splint, eating splint, finger rings for eating, brace, full fixation splint, partial fixation splint, after-surgery splint, emergency fixation splint, nasal splint, posterior lower leg splint, posterior full leg splint, posterior elbow splint, sugar tong splint, double sugar tong splint, long arm posterior splint, long arm fixation, wrist-elbow splint, carpal tunnel splint, ulnar gutter splint, wrist shield splint, joint shield splint, knee shield splint, knee conformer splint, elbow shield splint, basketball wrist splint, sports splint, foot rest splint, tendom splint, burns splint, foot drop splint, hand drop splint, airplane splint, general splint, tibia splint, radius fracture splint, ulna fracture splint, sprains splint, metacarpal fracture splint, sleeping ankle splint, flexible AFO, Anti-talus AFO, rigid AFO, tamarack flexure joint splint, foot orthosis, knee orthosis, spinal orthosis, cognitive disability splint, mental disability splint, sensation splint, prophylactic splint, functional and rehabilitation braces, fracture immobilizer, cervical thoracic orthosis (CTO), diabetic splint, braces, leg separation splint, shoe splint, toe plate extension splint, knee immobilizer, radial gutter splint, radial gutter fixation splint, protective collar splint, splints inserts, semi-rigid splint, soft splint, open palm splint and others.

In other embodiments, the prosthesis may be selected from a below elbow prosthetic, transracial, body-powered prosthesis, finger prosthesis, wrist prosthesis, arm prosthesis, forearm prosthesis, arm prosthesis, leg prosthesis, modular hand prosthesis, modular leg prosthesis, above-knee prosthesis, below-knee prosthesis, hip disarticulation prosthetic, prosthetic Feet, partial foot prosthetics, forequarter prosthesis, shoulder disarticulation, transhumeral prosthesis, elbow disarticulation prosthetic, transradial prosthesis, wrist disarticulation prosthetic, full hand prosthesis, partial finger prosthesis, passive prosthesis, externally powered prosthesis, sensation prosthesis, temperature prosthesis, wearable prosthesis, emergency prosthesis, work prosthesis, writing prosthesis, eating prosthesis, grasp prosthesis, pinch prosthesis, hook prosthesis, 2-fingers prosthesis, light prosthesis, splint-prosthesis, gross motor skills prosthesis, fine motor skills prosthesis, cooking prosthesis, showering prosthesis, bathing prosthesis, slipper prosthesis, night prosthesis, light prosthesis, home prosthesis, daily prosthesis, computer prosthesis, paralysis prosthesis, amyotrophic lateral sclerosis (ALS) prosthesis, polio prosthesis, multiple sclerosis (MS) prosthesis, below elbow prosthesis, above elbow prosthesis, bilateral prosthetic leg, knee disarticulation prosthesis, , Syme's prosthesis, toe prosthesis, trans-tibial prosthesis, trans-femoral prosthesis, robotic prosthetic, robotic arm, robotic leg, neurocognitive prosthesis, smart prosthesis, screen prosthesis, stroke prosthesis, traumatic brain injury prosthesis, cerebral palsy (CP) prosthesis, autism prosthesis, Alzheimer/AD prosthesis, memory prosthesis, ADL prosthesis, neural prosthetics, neuro prosthesis, Bio prosthesis, diabetic prosthesis, walking prosthesis, crutch-prosthesis, fit prosthesis, energy prosthesis, water prosthesis, beach prosthesis, bicycle prosthesis, bionic prosthesis, general prosthesis and others.

As will be explained in more details below, due to its properties, the device of this disclosure can also be used as a limb encasing device intended to function similarly to a cast. Thus, in some embodiments, the device may be a full fixation device, e.g. similar to a cast.

The ***limb*** may be an entire limb (e.g. an arm or a leg) or a portion thereof, such as a finger, a joint, a segment of bone, etc. It is also to be understood that the device may be designed for providing support to a human limb or for veterinary purposes (for example in treating various mammals, e.g. domestic, farm or wild animals).

The device is ***formable,*** hence is capable of being transformed from a first configuration to a second configuration by applying certain conditions (as will be described herein) and be formed into a desired shape in order to obtain a desired spatial geometry. As will also be explained in more details below, the device may be transformed from any first configuration (flat or bent) to any other second configuration, spatially differing from the first configuration. For example, the device may have a first flat configuration (e.g. in the form of a sheet), and is formed into a bent, 3 dimensional (3D) second configuration according to the anatomical features of the limb to be supported. Similarly, the first configuration may be a bent 3D shape, while the second configuration may be a different bent 3D shape, thus permitting re-using and re-customization of the device to a different patient once the device is no longer in use by a previous patient. In some embodiments, the device may be capable of undergoing at least 2, 3, 4, 5, 6, 7, 8, 9, 10 or even more cycles of transformation from a first configuration into the second configuration. Once fixed to a second configuration, and if it was reasonably used and for a period of no longer than a few years, the device may maintain its second configuration for a prolonged period of time, e.g. for at least about 2 years while still providing rigid orthopedic support.

Such formability, along with a desired strength and stability, is obtained by utilizing a thermoplastic polymeric material having a defined set of characteristics.

The ***thermoplastic polymer material*** is meant to refer to a polymeric material which is softened by heating to a state where it can be shaped, and once cooled below a threshold temperature, hardens to maintain its formed shape without undergoing a chemical reaction at the molecular level (i.e. the softening and the hardening of the thermoplastic polymer material is controlled only by thermal conditions and the material's thermal response, without involving breaking or forming chemical bonds between the polymer's molecules).

The term *polymer* (or polymeric material) includes homopolymers, copolymers, such as for example, block, graft, random and alternating copolymers as well as terpolymers, further including their derivatives, combinations and blends thereof. In addition to the above, the term includes all geometrical configurations of such structures including linear, block, graft, random, alternating, branched structures, and combinations thereof. The term *block copolymer* is meant to encompass a polymer formed from two or more homo-polymer subunits (blocks) linearly linked by chemical bonds (i.e. the blocks are connected end-to-end). Block copolymers with two, three, four and multiple homo-polymer units are referred to as di-block, tri-block, tetra-blocks and multi-blocks respectively. The number of monomer types in a block co-polymer may be less than or equal to the number of blocks. Thus, an ABC linear tri-block consists of three monomer types, whereas an ABA linear tri-block consists of two monomer types.

The thermoplastic polymeric material may be amorphous (namely, having no ordered structure of the polymer chains one with respect to the other), or semi-crystalline - including amorphous portions and crystalline (ordered) portions. According to some embodiments, the device is made of an amorphous thermoplastic polymeric material.

The devices of this disclosure are made of thermoplastic polymeric materials having a glass transition temperature (Tg) of between about 65°C and about 120°C. In some embodiments, the thermoplastic polymeric material may have a glass transition temperature (Tg) of the thermoplastic polymer material is between about 70°C and about 110°C. In other embodiments, the thermoplastic polymeric material may have a glass transition temperature (Tg) of the thermoplastic polymer material is between about 70°C and about 105°C. In some other embodiments, the thermoplastic polymeric material may have a glass transition temperature (Tg) of the thermoplastic polymer material is between about 80°C and about 110°C or even between about 80°C and about 105°C.

The ***glass transition temperature*** (Tg) is a characteristic temperature of a thermoplastic polymeric material, in which the amorphous portion of the polymer undergoes a reversible partial solid-to-liquid transition. Such transition causes softening of the polymeric material, allowing it to be shaped into the desired configuration. Once cooled to a temperature below the Tg, the polymeric material undergoes a liquid-to-solid transition, and thus by hardening and fixation of the shaped form.

As the devices of this disclosure are made of thermoplastic polymeric materials having a Tg of about 65°C and about 120°C, heating the device to the polymer's Tg does not require special heating equipment, and may be carried out by any method and apparatus that is capable of heating the device to the desired temperature. This is of particular importance for utilization of the device in disaster areas or third-world countries, where no access to sophisticated equipment is possible. For example, the device may be heated to its Tg to permit forming by merely immersing the device (in its first configuration) in boiling water - this is of importance as, in field conditions, the bubbling of boiling water is a straightforward visual indicator to a practitioner that a suitable temperature is obtained to permit immersion of the device.

Once the device needs to be re-shaped or re-formed, either due to a need to fine-tune the fit to the patient's anatomical shape or when requiring customizing the device to a different patient, a practitioner needs to merely heat the device to the Tg and the softening of the polymeric material enables reshaping of the device to another desired second configuration. Thus, the device of this disclosure is re-formable and re-usable by heating the device to the Tg of the polymeric material. The re-formation of the device for final repairs can be done either by immersing in boiling water, hot steams, heat gun or a blower.

In some embodiments, the polymeric material has a deflection temperature of at least 65°C at 0.455 MPa (when measured according to ISO 75), where the *deflection temperature* is a characteristic temperature to which the thermoplastic polymeric material needs to be heated in order to be shapeable and/or deformable. Namely, the device is made of a thermoplastic polymeric material that may be shapeable at a defined range of temperatures, in which the material is softened however does not yet undergo melting (i.e. complete phase change from solid to liquid). This enables the practitioner to form, mold or tailor the geometry and spatial configuration of the device (i.e. obtain the second configuration) in temperatures that may be obtained in field conditions, without utilizing specialized equipment, and by applying relatively low forces (e.g. shaping the device by hand).

In order to provide the desired support to the limb and maintain its second configuration, the thermoplastic polymeric material from which the device is made is required to have an elastic modulus of at least about 1500 MPa (mega-Pascal) and elongation to break of at least 75% (both measured according to ASTM D638). Such high elastic modulus and elongation to break enables the device to be hard on the one hand, however to also sufficiently absorb impact, thus providing mechanical support to the limb and absorbing possible impacts and loads without easily breaking.

For example, individuals after stroke/CVA who suffer from high muscle tone which causes a great deal of load on the wrist area, need rigid support to the wrist, palm and forearm. Without rigid support the device could either be significantly thickened, or bent or broken. High elastic modulus and elongation to break will enable them to have resistant-to-loads orthopedic support by a single-layered and lightweight device.

In some embodiments, the thermoplastic polymeric material has an elastic modulus of at least about 1600 MPa, at least about 1700 MPa, at least about 1800 MPa, at least about 1900 MPa, or even at least about 200 MPa. In other embodiments, the thermoplastic polymeric material has an elongation to break of at least about 80%, at least about 85%, at least about 90%, or even at least about 100%.

Such combination of features permits easy and fast shaping of the device in field conditions, however also results in a strong and durable device once hardened.

Non-limiting examples of suitable thermoplastic polymeric materials are thermoplastic polyurethanes, polyolefins (e.g. polypropylene, polypropylene), polypropylene terephthalate, polybutylene terephthalate, polyesters, co-polyester, etc., as well as blends and copolymers thereof.

In some embodiments, the thermoplastic polymeric material may be a polyester. In other embodiments, the thermoplastic polymeric material is glycol-modified polyethylene terephthalate (PETG).

In some embodiments, the thermoplastic polymeric material may be recyclable.

The devices of this disclosure may be made of polymeric materials that are transparent. Thus, in some embodiments the thermoplastic polymeric material may have a total light transmittance of at least 88% when measured according to ASTM D1003. In other embodiments, the thermoplastic polymeric material may have a total light transmittance of at least 89%, at least 90% or even at least 91% when measured according to ASTM D1003. Pressure sores are a common challenge with splinting and prosthetics. Wrong fixation, over fixation or localized pressure causing significant pain are common with fractures fixation by casting/plaster/splints. While most orthopedic devices are opaque, both pain and sores can be prevented if early visual detection of pressure is possible. Visual indication to a wrong fixation is usually lighter skin color on the pressure point and/or redness around the pressured area. The transparency of the device may be advantageous when the device is intended for closely contacting or fitting onto the limb. Such close fitting may often apply pressure on the tissue beneath the device, which may result in lack of comfort to the patient (and hence lack of compliance) at least, or even development of pressure-induced wounds and development of necrotic tissue in extreme situations. Thus, the transparency of the device may on the one hand help the practitioner to fine-tune the fitting of the device onto the limb during the shaping process, as well as assist the patient and the practitioner after fitting (and during the treatment duration) to identify development of undesired damage to the tissue.

However, it is also appreciated that the polymeric material may include pigments or other additives, as long as the properties of the thermoplastic polymeric material described herein remain substantively un-effected by such additional components.

As noted above, the device of this disclosure is formable and shapeable from a first spatial configuration to one or more second configurations when heated, while applying mild force to bend the device into the desired shape. Thus, the device of this disclosure may be further characterized by several geometrical features, each of which may exist in the device of this disclosure, alone or in various combinations.

The device may, in its first configuration, have a variable thickness profile along a cross-section taken perpendicular to a longitudinal axis of the device. In some embodiments, the thickness at the center of the profile may be larger compared to the thickness towards edges of the profile. Such variability in thickness may assist the practitioner to shape and form the edges of the device more accurately without substantially deforming the central segments of the device, such that the edges are bent to fit the patient's anatomical features, while the central segments of the device provide mechanical support due to their larger thickness.

Variability in thickness of the device also allows customization of the device to the limb's features (e.g. transverse carpal arch, one of the oblique arches) and fingers, thereby providing complete and more adapted support.

The device may have any suitable thickness that permits its shaping once heated, however, also provides sufficient mechanical support to the limb when used. Hand disability is often characterized with lowered-weight carrying ability. In many existing solutions, supporting devices are bulky and heavy and therefore are not used by patients. The common materials in the field (mainly polycaprolactone) are soft. As a result, therapists apply at least 2 layers of material for stronger support. This practice results in a heavy and thick device. Due to the thermoplastic polymeric material's mechanical properties, the devices of this disclosure may be thinner than similar devices made of other materials, thus providing the desired mechanical support by utilizing a thinner device (i.e. a lightweight device). In some embodiments, the device has a thickness of between about 1 mm and about 8 mm. In some embodiments, the device has a thickness of between about 1.5 mm and about 5 mm.

The device may also have an edge that defines a contour of the device, the edge being filleted to provide a smooth edge finish and prevent sharp edges from contacting the limb to prevent applying pressure on the tissue beneath the device. The fillets enable wearing the device without padding and allow it to remain transparent and with aeration. In some embodiments, the filleted edges have at least one portion with a curvature radius of between about 0.8 and about 2 mm.

The device may also comprise one or more through-holes that extend between a first face and a second, opposite face of the device. The through-holes provide for aeration to the tissue beneath the device, once applied onto the limb. The through-holes may be of any size and shape, may be arranged randomly along the device or in any ordered or semi-ordered array formation. In some embodiments, each of the through-holes may have a filleted hole edge, for example with a filleted hole edge having a curvature radius of between about 0.4 and about 2 mm.

As the device is made of thermoplastic polymeric material, the first configuration of the device may be obtained by injection molding of the thermoplastic polymeric material.

In order to allow fixation of the device, in its second configuration, onto the limb, the device may be configured with at least one slit or with at least one pair of slits, each slit being configured for receiving respective end portion of a fastening strip. The slits may be formed adjacent at a rim section of the device, thus enabling fixing an end of a fastening strip in each slit. It is, however, also contemplated within the scope of this disclosure that one or more slits may be formed in central portions of the device. Each fastening strip can be made of neoprene with hook and loop Velcro^{®} connectors for connecting it to its respective slit, or with Velcro^{®} alone. The strips can be easily disconnected from the device, and replaced with other (e.g., new) strips. In addition, the slits of the device can be easily cleaned, for example, for ongoing maintenance or when the splint is reused and/or reshaped for another patient.

In another aspect, this disclosure provides a method of forming an orthopedic device, comprising:
- heating a device having a first configuration to a temperature of between about 65°C and about 140°C, thus obtaining a malleable pre-formed device, the device being made of a thermoplastic polymeric material selected to have an elastic modulus of at least about 1500 MPa when measured according to ASTM D638, a glass transition temperature (Tg) of between about 65°C and about 120°C, and elongation to break of at least 75% when measured according to ASTM D638;
- bringing the malleable pre-formed device into direct or indirect contact with a body limb requiring support;
- forming the malleable pre-formed device into a second configuration matching the form of said limb thus obtaining a malleable formed device;
- permitting the malleable formed device to cool, thus obtaining said orthopedic device.

At a first stage, the device, in its first configuration, is heated to a defined temperature (i.e. at least to its Tg temperature, however below its melting temperature) for a period of time to allow it to soften, however without melting. The heating permits obtaining a *malleable* state of the device, such that relatively low forces, such as forces applied by the practitioner fitting the device, are sufficient in order to shape the device into its second configuration. Heating the device, when at its first configuration, to the desired temperature may be carried out by immersing in boiling water, or heating by hot air. As noted above, in some embodiments, heating is carried out by immersing the device in boiling water such that the bubbling of boiling water is used as a visual indicator that the desired temperature has been obtained without utilizing any temperature measurement equipment. In some embodiments, the device is heated to a temperature of between about 70°C and about 110°C, or even to a temperature of between about 70°C and about 105°C.

Exposure may be carried out for a period of time of at least 20 seconds, at least 30 seconds, at least 40 seconds, at least 50 seconds or even at least about 60 seconds; in some embodiments, the device is heated for a period of time of between about 20 seconds and 5 minutes (or even for a period of time between about 20 seconds and 3 minutes).

Once the device is in its pre-formed malleable state, the device is brought into contact with the limb to be supported, and the practitioner can apply a force on various areas of the device in order to fit the device to the anatomical shape of the limb to be supported, obtaining a malleable formed device. In cases of difficulty to adjust according to the injured limb, such as in fractures or limb deformities, the practitioner can adjust the device on its own limb and apply changes and repairs while checking the formed shape on the patient's limb. After fitting is complete, the device is left to cool, thereby hardening and setting the device in its second configuration to obtain the orthopedic device.

In some embodiments, the malleable formed device is permitted to cool to room temperature (e.g. 15-40°C) within about 25 seconds to 3 minutes. In other embodiments, cooling within about 25 seconds to 2 minutes, or even within about 25 seconds to 1 minute, may be sufficient to set the device in its second configuration.

The device is formed from its first configuration to its second configuration while in said Tg temperature. In order to prevent damage to the limb when shaping the device, indirectly contacting the malleable pre-formed device with the body limb may include at least partially wrapping the body limb requiring support with a thermally protective sheet (such as thick fabric or thick glove) closely following the contours of the body limb, and bringing the malleable pre-formed device into contact with the protective layer. Namely, the limb is being first wrapped by the protective sheet, and as the sheet follows the contours of the limb, shaping of the device may be carried out by fitting the pre-formed malleable device over the protective sheet.

The device may be provided in its first configuration in a shape and dimension that correspond to the shape of the limb to be treated. Thus, utilizing the devices and methods of this disclosure, the entire forming process of the orthopedic device (from the device's first configuration until obtaining the second, hardened configuration) is relatively short and simple, taking often between about 2 and about 5 minutes.

In another one of its aspects, this disclosure provides a kit comprising a first device as described herein and at least one fastening strip, the first device being configured for receiving said at least one fastening strip.

The kit may further comprise at least one second device as described herein, configured for receiving said at least one fastening strip, said first and second devices being connected to one another by the fastening strips for encasing a body limb in need of support. Such kits may be used to fit two or more devices of this disclosure around a limb to be supported, either to provide segmented support or to encase the limb by supporting devices (obtaining a supporting fixation similar to that obtained by a cast). In another example, the kit may include a first device, and a plurality of second devices configured to interconnect with the first device by a plurality of strips.

In another example, the kit may include at least 2 devices, configured to interconnect by strips and/or other connectors.

A further example is a kit including 2 or more parts of a prosthesis device as described herein and means of attaching them one to the other. Therefore, in some embodiments, the kit may comprise at least one first prosthesis device and at least one second prosthesis device, and being configured for association with one another to form a prosthesis device (e.g. by fastening strips).

The kit may further comprise at least one of at least one heat-insulating glove and a thermally protective sheet, as well as instructions for use.

As used herein, the term ***about*** is meant to encompass deviation of ±10% from the specifically mentioned value of a parameter, such as temperature, pressure, concentration, etc.

Whenever a numerical range is indicated herein, it is meant to include any cited numeral (fractional or integral) within the indicated range. The phrases *"****ranging*/*****ranges between**"* a first indicated number and a second indicated number and "ranging/ranges from" a first indicated number "to" a second indicated number are used herein interchangeably and are meant to include the first and second indicated numbers and all the fractional and integral numerals therebetween.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1A** is a perspective view of the orthopedic device in the form of a splint, according to one embodiment of the presently disclosed subject matter, in which the splint is in its first flat configuration;
**Fig. 1B** is a front, top and side views of the splint of Fig. 1A;
**Fig. 1C** is a perspective view of the splint of Fig. 1A, in which the splint is in its second bent configuration;
**Fig. 1D** is a perspective view of the splint of Fig. 1A, in which the splint is in its third bent configuration; and
**Fig. 2** is a schematic illustration of method of using a kit including the splint of Fig. 1A, and in particular of forming the splint.

### DETAILED DESCRIPTION OF EMBODIMENTS

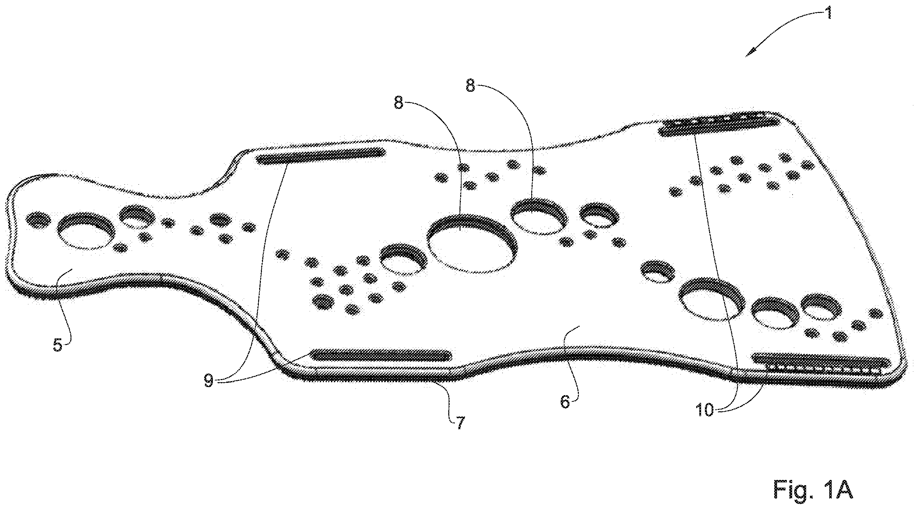

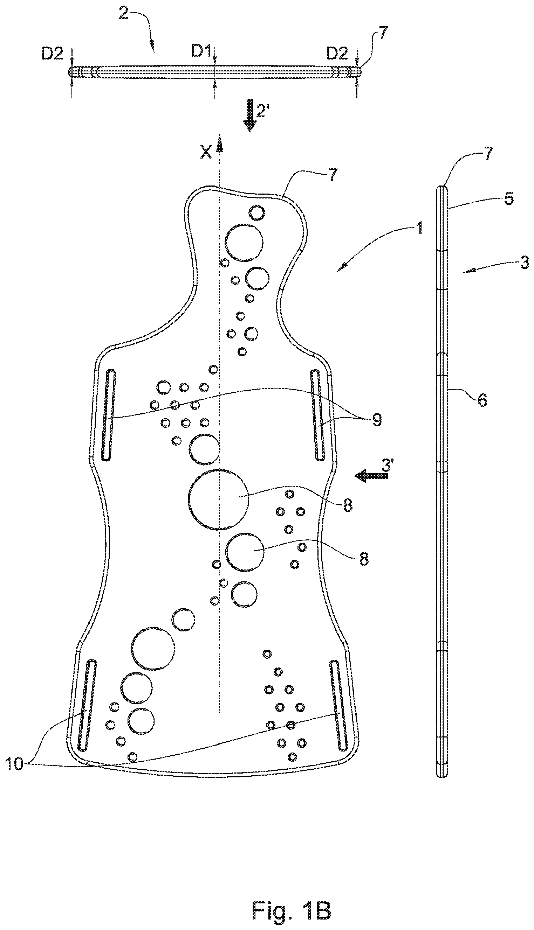

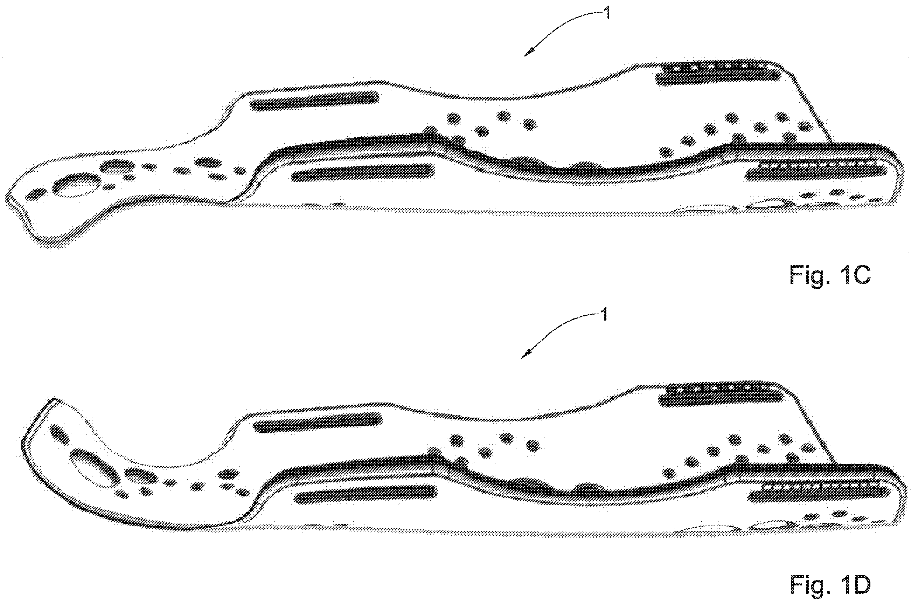

Reference is now made to Figs. 1A-1D, illustrating one example of an orthopedic device according to the presently disclosed subject matter, in the form of a functional volar splint, generally designated as 1. The splint 1 in Figs. 1A and 1B is shown in its first flat configuration, in Fig. 1C in its second bent configuration, and in Fig. 1D in its third bent configuration. The splint 1 is shaped to fit a patient's forearm and wrist in order to provide support to the wrist, for example, in the second bent configuration. The splint 1 according to the particular example of Figs. 1A-1D is made of glycol-modified polyethylene terephthalate (PETG), having the following characteristics: a Tg of between about 90°C and about 105°C, an elastic modulus of about 2200 MPa when measured according to ASTM D638, an elongation to break of about 150% when measured according to ASTM D638, and a total light transmittance of about 91% when measured according to ASTM D1003. The splint 1 is pre-shaped by injection molding.

The splint 1 is configured to be easily and quickly tailored to the patient's anatomical features, without using expensive equipment and without requiring specialization in splinting for properly fitting the splint to the patient. Further, the splint 1 is mechanically stable, such that once shaped and customized into the desired spatial configuration, e.g., the second bent configuration and the third bent configuration, and hardened, the splint 1 provides proper support to the injured limb. Further, the splint 1 is formable and shapeable without the need of specialized equipment, thus making the splint 1 suitable for providing treatment to populations in poor or developing countries, as well as in disaster areas. The material of the splint 1 allows heating it to its polymer's Tg without using special heating equipment, and may be carried out by boiling water to the desired temperature. This is of particular importance for utilization of the splint 1 in disaster areas or developing countries, where no access to sophisticated equipment is available. For example, the splint 1 may be heated to its Tg to permit forming by merely immersing the splint 1 (in its first configuration) in a boiling water pot including water with a temperature of about 100°C. This is of importance as, in field conditions, the bubbling of boiling water is a straightforward visual indicator to a practitioner that a suitable temperature is obtained to permit immersion of the splint 1.

The third bent configuration of Fig. 1D is provided in the drawings, to illustrate how the splint 1 can be reshaped from the second bent configuration of Fig. 1C to a different bent 3D shape, thus permitting reusing and retailoring the splint 1 to a different patient once the splint 1 is no longer in use by a previous patient.

Such formability, along with a desired strength and stability, is obtained by utilizing the above described thermoplastic polymeric material of splint 1 having the above mentioned defined set of characteristics.

Once the splint 1 needs to be reshaped or reformed, either due to a need to fine-tune the fit to the patient's anatomical shape or when requiring adjusting the splint 1 to a different patient, a practitioner needs to merely heat the splint 1 to the Tg and the softening of the polymeric material enables reshaping of the splint 1 to another desired second configuration (e.g., the second bent configuration of Fig. 1C, or the third bent configuration of Fig. 1D).

The transparency of the splint 1 is advantageous when the splint 1 is intended for closely contacting or fitting onto the limb. Such close fitting may often apply pressure on the tissue beneath the splint 1, which may result in lack of comfort to the patient (and hence lack of compliance) at least, or even development of pressure-sores and development of necrotic tissue in a different extreme situation. Thus, the transparency of the splint 1 may on the one hand help the practitioner to fine tune the fitting of the splint 1 onto the limb during the shaping process, as well as assist the patient and the practitioner after fitting (and during treatment) to identify development of undesired damage to the tissue.

Reference is now made particularly to Fig. 1B, and in which the splint 1 is shown from its front view, from its top view 2 when viewed from direction 2' (above the splint 1), and from its side view 3 when viewed from direction 3' (right to the splint 1).

As clearly seen in top view 2, the splint 1 has a variable thickness profile along a cross-section (not shown) taken perpendicularly to the longitudinal axis X of the splint 1, and being parallel to top view 2 of the splint 1. The variable thickness profile seen in the top view 2 is expressed by a thickness D1 at the center of the profile, which is larger compared to a thickness D2 at the edges of the profile. Such variability in thickness may assist the practitioner to shape and form the edges of the splint 1 more accurately without substantially deforming the central segments of the splint 1, such that the edges are bent to fit the patient's anatomical features, while the central segments of the splint 1 provide mechanical support due to their larger thickness.

As seen in Figs. 1A and 1B, the splint 1 has a filleted edge 7 that defines a contour thereof. The filleted edge 7 is configured to provide a smooth edge finish and prevent sharp edges from contacting the limb to prevent applying pressure on the tissue beneath the splint 1. The filleted edge 7 has a curvature radius of about 1.5 mm.

The splint 1 also has a plurality of through-holes 8 that extend between a first face and a second, opposite face of the splint 1. The through-holes 8 provide for aeration to the tissue beneath the splint 1, one applied onto the limb. The through-holes 8 have varying sizes and are arranged as a semi-ordered array formation. Each of the through-holes 8 also has a filleted hole edge, with a curvature radius of about 0.5 mm.

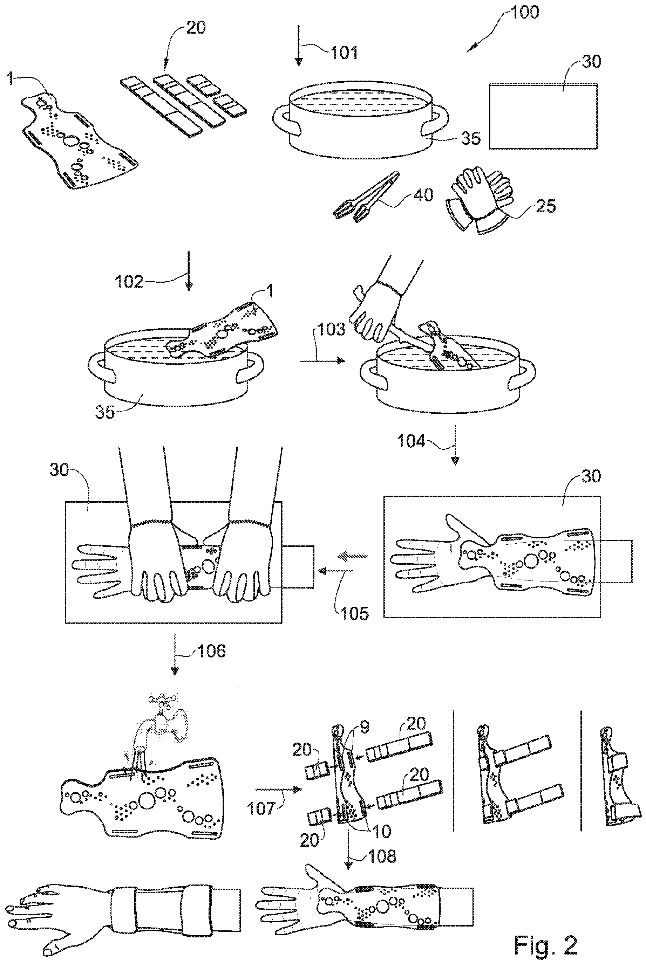

In order to allow customization of the splint 1, in its second bent configuration, onto the forearm, the lower portion 6 of the splint 1 is configured with two pairs of slits 9 and 10. Each of the slits 9 and 10 extends in proximity to the edge 7 has an elongate structure configured for receiving a respective end portion of a fastening strip 20 (as detailed below with respect to Fig. 2). Each fastening strip 20 can be made with or without Neoprene, and with hook and loop Velcro connectors for connecting the strip 20 to its respective slit 9 and 10. The strips 20 can be disconnected from the splint 1, are replaced with other (e.g., new) strips 20. In addition, the slits 9 and 10 of the splint 1 can be easily cleaned, for example, during usual maintenance or when the splint 1 is reused for another patient.

Reference is now made to Fig. 2, in which a kit 100 including the splint 1 of Fig. 1A, is shown together with illustration of method steps for using the kit 100.

The kit 100 includes the following elements: the splint 1, four fastening strips 20, a pair of heat-insulating gloves 25, a thermally protective sheet 30, a pot 35 for boiling water, tongs 40.

As shown in Fig. 2, the method of using the kit 100 for forming the splint 1 comprises the following steps:
- step 101: providing the kit 100, including the splint 1 in its first flat configuration;
- step 102: plunging the splint 1 in its first flat configuration into the pot 35 and exposing the splint 1 to a temperature of about 100°C;
- step 103: waiting for a period of time of about 50 seconds to soften the splint 1, however without melting, thus obtaining a malleable pre-formed splint 1, and extracting the malleable pre-formed splint 1 from the pot 35;
- step 104: wrapping a patient's forearm and wrist requiring support with the thermally protective sheet 30 and bringing the malleable pre-formed splint 1 into contact with the patient's forearm and wrist;
- step 105: application force on various areas of the splint 1 in order to fit the splint 1 to the anatomical shape of the patient's forearm and wrist, and thereby forming the malleable pre-formed splint 1 into a second configuration matching the form of said forearm and wrist, and obtaining a malleable formed splint 1;
- step 106: permitting the malleable formed splint 1 to cool to room temperature of about 24°C along about 3 minutes, for example, by using cold water, thus obtaining said splint 1 in its second bent configuration;
- step 107: introducing each one of the strips 20 to their respective slits 9 and 10, and connecting the hook and loop Velcro connectors of each of the strips, thereby obtaining the splint 1 in its second bent configuration ready for use; and
- step 108: attaching the splint 1 to patient's forearm, for providing support to his wrist, and observing the fixed area through the transparent splint 1 for receiving visual indication to pressure points and for correcting the customization of the splint 1 to the pressured area at the point of care.

As is clear from the above description, utilizing the kit 100 and the above method, the entire process of forming the splint 1 from its first flat configuration until obtaining the second bent configuration is relatively short and simple, taking often up to 5 minutes.

Although not shown in the drawings, the kit 100 can further comprise a splint configured to be connected to the splint 1 by fastening strips for encasing a body limb in need of support. Such kits may be used to fit two or more splints of this disclosure around a limb to be supported, either to provide segmented support or to encase the limb by supporting splints (obtaining a fixation similar to that obtained by a cast).

## Claims

1. A formable orthopedic device (1) having at least one first configuration and at least one second configuration, the second configuration being adapted to provide support to at least one limb, the device being made of a thermoplastic polymeric material having a glass transition temperature (Tg) of between 65°C and 120°C, the device being capable to be formed into the second configuration by heating the device to said Tg, **characterized in that** the thermoplastic polymeric material is selected to have an elastic modulus of at least 1500 MPa when measured according to ASTM D638-14 (15 December 2014) and elongation to break of at least 75% when measured according to ASTM D638-14 (15 December 2014), the device being a single layered transparent orthopedic device being re-formable upon heating to the Tg.

2. The device of claim 1, wherein the glass transition temperature (Tg) of the thermoplastic polymer material is between 70°C and 105°C.

3. The device of any one of claims 1 to 2, wherein the thermoplastic polymeric material is recyclable.

4. The device of any one of claims 1 to 3, wherein the thermoplastic polymeric material is amorphous or semi-crystalline.

5. The device of any one of claims 1 to 4, wherein the thermoplastic polymeric material has a total light transmittance of at least 88% when measured according to ASTM D1003-13 (15 November 2013).

6. The device of any one of claims 1 to 5, wherein the deflection temperature of the thermoplastic material is at least 65°C at 0.455 MPa when measured according to ISO 75-1 or ISO 75-2 (2013).

7. The device of any one of claims 1 to 6, wherein the thermoplastic polymeric material is selected from polyesters, thermoplastic polyurethanes, and blends and copolymers thereof.

8. The device of claim 7, wherein the thermoplastic polymeric material is glycol-modified polyethylene terephthalate (PETG).

9. The device of any one of claims 1 to 8, wherein the first configuration of the device is a planar formed sheet and the second configuration of the device is a non-planar 3-dimensional device.

10. The device of any one of claims 1 to 9, being a splint (1).

11. A method of forming an orthopedic device, **characterized by**:
obtaining a first configuration of a single layered transparent orthopedic device made of a thermoplastic polymeric material;
heating the device having the first configuration to a temperature of between 65°C and 140°C, thus obtaining a malleable pre-formed device, the device being made of a thermoplastic polymeric material selected to have an elastic modulus of at least 1500 MPa when measured according to ASTM D638-14 (15 December 2014), a glass transition temperature (Tg) of between 65°C and 120°C, and elongation to break of at least 75% when measured according to ASTM D638-14 (15 December 2014);
bringing the malleable pre-formed device into direct or indirect contact with a body limb requiring support;
forming the malleable pre-formed device into a second configuration matching the form of said limb thus obtaining a malleable formed device;
permitting the malleable formed device to cool and harden, thus obtaining said orthopedic device,
wherein the orthopedic device is re-formable upon heating to the Tg.

12. The method of claim 11, wherein said heating is carried out by immersing the device in boiling water.

13. The method of any one of claims 11 and 12, wherein indirect contacting the malleable pre-formed device with the body limb includes at least partially wrapping the body limb requiring support with a thermally protective sheet (30) closely following the contours of the body limb, and bringing the malleable pre-formed device into contact with the protective layer (30).

14. A kit comprising a first device (1) according to any one of claims 1 to 10 and at least one fastening strip (20), the first device (1) being configured for receiving said at least one fastening strip.

## Patentansprüche

1. Verformbare orthopädische Vorrichtung (1) mit mindestens einer ersten Konfiguration und mindestens einer zweiten Konfiguration, wobei die zweite Konfiguration geeignet ist, mindestens eine Gliedmaße zu stützen, wobei die Vorrichtung aus einem thermoplastischen Polymermaterial mit einer Glasübergangstemperatur (Tg) zwischen 65°C und 120°C hergestellt ist, wobei die Vorrichtung durch Erhitzen der Vorrichtung auf die Tg in die zweite Konfiguration geformt werden kann,
**dadurch gekennzeichnet, dass** das thermoplastische Polymermaterial so ausgewählt ist, dass es einen Elastizitätsmodul von mindestens 1500 MPa, gemessen nach ASTM D638-14 (15. Dezember 2014), und eine Bruchdehnung von mindestens 75%, gemessen nach ASTM D638-14 (15. Dezember 2014), aufweist,
wobei die Vorrichtung eine einschichtige transparente orthopädische Vorrichtung ist, die bei Erwärmung auf die Tg wieder verformbar ist.

2. Vorrichtung nach Anspruch 1, wobei die Glasübergangstemperatur (Tg) des thermoplastischen Polymermaterials zwischen 70°C und 105°C liegt.

3. Vorrichtung nach einem der Ansprüche 1 bis 2, wobei das thermoplastische polymere Material recycelbar ist.

4. Vorrichtung nach einem der Ansprüche 1 bis 3, wobei das thermoplastische polymere Material amorph oder teilkristallin ist.

5. Vorrichtung nach einem der Ansprüche 1 bis 4, wobei das thermoplastische polymere Material eine Gesamtlichtdurchlässigkeit von mindestens 88 % aufweist, gemessen nach ASTM D1003-13 (15. November 2013).

6. Vorrichtung nach einem der Ansprüche 1 bis 5, wobei die Verformungstemperatur des thermoplastischen Materials mindestens 65 °C bei 0,455 MPa beträgt, gemessen nach ISO 75-1 oder ISO 75-2 (2013).

7. Vorrichtung nach einem der Ansprüche 1 bis 6, wobei das thermoplastische Polymermaterial aus Polyestern, thermoplastischen Polyurethanen und deren Mischungen und Copolymeren ausgewählt ist.

8. Vorrichtung nach Anspruch 7, wobei das thermoplastische Polymermaterial Glykol-modifiziertes Polyethylenterephthalat (PETG) ist.

9. Vorrichtung nach einem der Ansprüche 1 bis 8, wobei die erste Konfiguration der Vorrichtung eine planar geformte Schicht ist und die zweite Konfiguration der Vorrichtung eine nicht planare dreidimensionale Vorrichtung ist.

10. Vorrichtung nach einem der Ansprüche 1 bis 9, die eine Schiene (1) ist.

11. Verfahren zur Herstellung einer orthopädischen Vorrichtung, **gekennzeichnet durch**:
Erhalten einer ersten Konfiguration einer einschichtigen transparenten orthopädischen Vorrichtung aus einem thermoplastischen Polymermaterial;
Erhitzen der Vorrichtung mit der ersten Konfiguration auf eine Temperatur zwischen 65°C und 140°C, wodurch eine verformbare vorgeformte Vorrichtung erhalten wird, wobei die Vorrichtung aus einem thermoplastischen Polymermaterial hergestellt ist, das so ausgewählt ist, dass es einen Elastizitätsmodul von mindestens 1500 MPa, gemessen nach ASTM D638-14 (15. Dezember 2014), eine Glasübergangstemperatur (Tg) zwischen 65°C und 120°C und eine Bruchdehnung von mindestens 75%, gemessen nach ASTM D638-14 (15. Dezember 2014), aufweist;
Bringen der verformbaren vorgeformten Vorrichtung in direkten oder indirekten Kontakt mit einer Gliedmaße, die Unterstützung benötigt;
Formen der verformbaren vorgeformten Vorrichtung in eine zweite Konfiguration, die der Form der Gliedmaße entspricht, wodurch eine verformbare geformte Vorrichtung erhalten wird;
Abkühlen lassen und Aushärten der verformbaren, geformten Vorrichtung, um die orthopädische Vorrichtung zu erhalten,
wobei die orthopädische Vorrichtung bei Erwärmung auf die Tg wieder verformbar ist.

12. Verfahren nach Anspruch 11, wobei das Erhitzen durch Eintauchen der Vorrichtung in kochendes Wasser erfolgt.

13. Verfahren nach einem der Ansprüche 11 und 12, wobei das indirekte Inkontaktbringen der verformbaren vorgeformten Vorrichtung mit der Gliedmaße das zumindest teilweise Umhüllen der zu stützenden Gliedmaße mit einer Wärmeschutzschicht (30), die den Konturen der Gliedmaße eng folgt, und das Inkontaktbringen der verformbaren vorgeformten Vorrichtung mit der Schutzschicht (30) umfasst.

14. Kit, umfassend eine erste Vorrichtung (1) nach einem der Ansprüche 1 bis 10 und mindestens einen Befestigungsstreifen (20), wobei die erste Vorrichtung (1) zur Aufnahme des mindestens einen Befestigungsstreifens ausgebildet ist.

## Revendications

1. Dispositif orthopédique pouvant être mis en forme (1) ayant au moins une première configuration et au moins une seconde configuration, la seconde configuration étant adaptée pour fournir un support à au moins un membre, le dispositif étant fait d'un matériau polymère thermoplastique ayant une température de transition vitreuse (Tg) entre 65°C et 120°C, le dispositif étant capable d'être mis en forme dans la seconde configuration par chauffage du dispositif à ladite Tg,
**caractérisé en ce que** le matériau polymère thermoplastique est choisi pour avoir un module d'élasticité d'au moins 1500 MPa lorsqu'il est mesuré selon ASTM D638-14 (15 décembre 2014) et un allongement à la rupture d'au moins 75 % lorsqu'il est mesuré selon ASTM D638-14 (15 décembre 2014),
le dispositif étant un dispositif orthopédique transparent à une seule couche pouvant être remis en forme par chauffage à la Tg.

2. Dispositif selon la revendication 1, dans lequel la température de transition vitreuse (Tg) du matériau polymère thermoplastique est entre 70°C et 105°C.

3. Dispositif selon l'une quelconque des revendications 1 à 2, dans lequel le matériau polymère thermoplastique est recyclable.

4. Dispositif selon l'une quelconque des revendications 1 à 3, dans lequel le matériau polymère thermoplastique est amorphe ou semi-cristallin.

5. Dispositif selon l'une quelconque des revendications 1 à 4, dans lequel le matériau polymère thermoplastique a une transmittance lumineuse totale d'au moins 88 % lorsqu'elle est mesurée selon ASTM D1003-13 (15 novembre 2013).

6. Dispositif selon l'une quelconque des revendications 1 à 5, dans lequel la température de fléchissement du matériau thermoplastique est d'au moins 65°C à 0,455 MPa lorsqu'elle est mesurée selon ISO 75-1 ou ISO 75-2 (2013).

7. Dispositif selon l'une quelconque des revendications 1 à 6, dans lequel le matériau polymère thermoplastique est choisi parmi les polyesters, les polyuréthanes thermoplastiques, et leurs mélanges et copolymères.

8. Dispositif selon la revendication 7, dans lequel le matériau polymère thermoplastique est du polyéthylène téréphtalate modifié par glycol (PETG).

9. Dispositif selon l'une quelconque des revendications 1 à 8, dans lequel la première configuration du dispositif est une feuille mise en forme plane et la seconde configuration du dispositif est un dispositif tridimensionnel non plan.

10. Dispositif selon l'une quelconque des revendications 1 à 9, étant une attelle (1).

11. Procédé de mise en forme d'un dispositif orthopédique, **caractérisé par**:
l'obtention d'une première configuration d'un dispositif orthopédique transparent à une seule couche fait d'un matériau polymère thermoplastique;
le chauffage du dispositif ayant la première configuration à une température entre 65°C et 140°C, obtenant ainsi un dispositif prémis en forme malléable, le dispositif étant fait d'un matériau polymère thermoplastique choisi pour avoir un module d'élasticité d'au moins 1500 MPa lorsqu'il est mesuré selon ASTM D638-14 (15 décembre 2014), une température de transition vitreuse (Tg) entre 65°C et 120°C, et un allongement à la rupture d'au moins 75 % lorsqu'il est mesuré selon ASTM D638-14 (15 décembre 2014);
la mise en contact directe ou indirecte du dispositif prémis en forme malléable avec un membre du corps nécessitant un support;
la mise en forme du dispositif prémis en forme malléable dans une seconde configuration correspondant à la forme dudit membre, obtenant ainsi un dispositif mis en forme malléable;
le refroidissement et le durcissement du dispositif mis en forme malléable, obtenant ainsi ledit dispositif orthopédique,
dans lequel le dispositif orthopédique peut être remis en forme par chauffage à la Tg.

12. Procédé selon la revendication 11, dans lequel ledit chauffage est effectué par immersion du dispositif dans l'eau bouillante.

13. Procédé selon l'une quelconque des revendications 11 et 12, dans lequel la mise en contact indirecte du dispositif prémis en forme malléable avec le membre du corps inclut l'enveloppement au moins partiel du membre du corps nécessitant un support avec une feuille de protection thermique (30) suivant étroitement les contours du membre du corps, et la mise en contact du dispositif prémis en forme malléable avec la couche de protection (30).

14. Kit comprenant un premier dispositif (1) selon l'une quelconque des revendications 1 à 10 et au moins une sangle de fixation (20), le premier dispositif (1) étant configuré pour recevoir ladite au moins une sangle de fixation.
